# EUROPEAN PATENT APPLICATION

(11) **EP 4 553 165 A1**
(43) Date of publication of application: **14.05.2025**
(21) Application number: 23863180.8
(22) Date of filing: 05.09.2023
(51) Int. Cl.: C12Q 1/04, C12N 15/31, C12N 15/60, C12Q 1/6844, C12Q 1/6876, C12Q 1/689

(54) **METHOD FOR DETECTING FUSOBACTERIUM NUCLEATUM AND METHOD FOR DETECTING FUSOBACTERIUM NUCLEATUM NUCLEATUM**

(30) Priority: 06.09.2022 JP 2022141680
(71) Applicant: EIKEN KAGAKU KABUSHIKI KAISHA, Tokyo 110-8408 (JP)
(72) Inventor: KAKU Toshisuke, Shimotsuga-gun, Tochigi 329-0114 (JP)
(74) Representative: Boult Wade Tennant LLP
(86) International application number: PCT/JP2023/032374
(87) International publication number: WO 2024/053642

(57) **Abstract**

According to one aspect of the present invention, this method for detecting *Fusobacterium nucleatum* in a sample involves a step for subjecting a DNA derived from a sample to nucleic acid amplification reaction using a primer set specific to a citrate lyase beta subunit gene of *Fusobacterium nucleatum.* According to said method, *F. nucleatum* can be specifically detected. According to another aspect of the present invention, this method for detecting *Fusobacterium nucleatum nucleatum* in a sample involves a step for subjecting a DNA derived from a sample to nucleic acid amplification reaction using a primer set specific to a dicarboxylate/amino acid: cation cotransporter gene of *Fusobacterium nucleatum nucleatum.* According to said method, *F*. *nucleatum nucleatum* can be specifically detected.

## Description

### Technical Field

The present invention relates to a method for detecting *Fusobacterium nucleatum* and a method for detecting *Fusobacterium nucleatum subsp. nucleatum.*

### Background Art

*Fusobacterium nucleatum (F. nucleatum)* is a bacterium living in the human oral cavity and colon. *F. nucleatum* is an anaerobic bacterium that has recently attracted attention as a bacterium associated with various diseases, such as colorectal cancer and periodontal disease. There are four subspecies of the bacterium: *F. nucleatum subsp. animalis, F. nucleatum subsp. polymorphum, F. nucleatum subsp. nucleatum,* and *F. nucleatum subsp. vincentii.*

As a method for detecting bacteria, a method for detecting 16S rRNA of bacteria is known. For example, in an Example of Patent Literature 1, *F. nucleatum* is detected using a probe for 16S rRNA of *Fusobacterium.* However, this method poses a problem in specificity, and in the Example of Patent Literature 1, probes for 16S rRNA of *Fusobacterium* have been shown to cross-react with various bacteria including Campylobacter.

As a method for identifying *F. nucleatum* in a sample at the bacterial strain level, which is an even finer classification than the subspecies, there is a method including performing PCR using a combination of primers capable of amplifying a DNA containing a CRISPR region to detect the presence or absence of a CRISPR-Cas sequence containing the CRISPR region and the length of the sequence (Patent Literature 2). However, the detection of the presence or absence of a CRISPR-CAS sequence and the length of the sequence requires electrophoresis or nucleotide sequence determination of PCR products, and such a method poses a practical problem in terms of simplicity. In addition, such a method also poses a practical problem in terms of detection sensitivity.

On the other hand, there is no known method for broadly and specifically detecting the four subspecies or specific subspecies of *F*. *nucleatum* without identification at the strain level.

### Citation List

### Patent Literature

| | |
|---|---|
| Patent Literature 1: | Japanese Patent No. 7040562 |
| Patent Literature 2: | Japanese Patent No. 6945294 |

### Summary of Invention

### Technical Problem

An object of the present invention is to specifically detect *F*. *nucleatum.* In addition, another object of the present invention is to specifically detect *F*. *nucleatum subsp. nucleatum.*

### Solution to Problem

In order to find a species-specific sequence and a subspecies-specific sequence of *Fusobacterium,* it is necessary to analyze databases of the National Center for Biotechnology Information (NCBI) and the like. However, it would be very difficult to search for a specific sequence across the entire 2000 kbp genome of the *Fusobacterium* bacteria from approximately 50,000 sequence information, and there are no reported examples of such analyses. Given these circumstances, no common sequence has been found for species or subspecies of *Fusobacterium,* other than the 16S rRNA gene and CRISPR.

On the other hand, as described below, as a result of extensive studies, the present inventor has succeeded in analyzing a huge amount of sequence information and found a gene with a species-specific sequence from the entire genome of *F*. *nucleatum.* Then, the present inventor has found that *F*. *nucleatum* could be specifically detected by using a primer set for a loop-mediated isothermal amplification (LAMP) reaction prepared based on the nucleotide sequence of such a gene. In addition, as described below, as a result of extensive studies, the present inventor has found a gene with a subspecies-specific nucleotide sequence from the entire genome of *F*. *nucleatum subsp. nucleatum.* Then, the inventor has found that *F*. *nucleatum subsp. nucleatum* can be specifically detected by using a primer set for a LAMP reaction prepared based on the nucleotide sequence of such a gene. The present invention is a revolutionary invention completed based on these findings of the present inventor.

Namely, the present invention has the following aspects.
[1] A method for detecting *Fusobacterium nucleatum* in a sample, comprising:
   a step of performing a nucleic acid amplification reaction on a DNA derived from the sample using a primer set specific to a citrate lyase beta subunit gene of *Fusobacterium nucleatum.*
[2] The method according to [1], wherein the citrate lyase beta subunit gene consists of the nucleotide sequence set forth in any one of SEQ ID NOs: 1 to 4 or a nucleotide sequence having 90% or more sequence identity to the nucleotide sequence set forth in any one of SEQ ID NOs: 1 to 4.
[3] The method according to [2], wherein the primer set is a primer set specific to
   a region from a 141st to a 415th base,
   a region from a 4th to a 253rd base,
   a region from a 238th to a 561st base,
   a region from a 577th to a 806th base, or
   a region from a 32nd to a 636th base in the nucleotide sequence set forth in any one of SEQ ID NOs: 1 to 4,
   or a region having 90% or more sequence identity to any of these regions.
[4] The method according to any one of [1] to [3], wherein the nucleic acid amplification reaction is a loop-mediated isothermal amplification reaction or a polymerase chain reaction.
[5] The method according to [4],
   wherein the nucleic acid amplification reaction is a loop-mediated isothermal amplification reaction, and
   wherein the primer set is
   (a1) a primer set comprising an F3 primer consisting of the nucleotide sequence set forth in SEQ ID NO: 5, a B3 primer consisting of the nucleotide sequence set forth in SEQ ID NO: 6, an FIP primer consisting of the nucleotide sequence set forth in SEQ ID NO: 7, and a BIP primer consisting of the nucleotide sequence set forth in SEQ ID NO: 8,
   (a2) a primer set comprising an F3 primer consisting of the nucleotide sequence set forth in SEQ ID NO: 31, a B3 primer consisting of the nucleotide sequence set forth in SEQ ID NO: 32, an FIP primer consisting of the nucleotide sequence set forth in SEQ ID NO: 33, and a BIP primer consisting of the nucleotide sequence set forth in SEQ ID NO: 34,
   (a3) a primer set comprising an F3 primer consisting of the nucleotide sequence set forth in SEQ ID NO: 37, a B3 primer consisting of the nucleotide sequence set forth in SEQ ID NO: 38, an FIP primer consisting of the nucleotide sequence set forth in SEQ ID NO: 39, and a BIP primer consisting of the nucleotide sequence set forth in SEQ ID NO: 40, or
   (a4) a primer set comprising an F3 primer consisting of the nucleotide sequence set forth in SEQ ID NO: 43, a B3 primer consisting of the nucleotide sequence set forth in SEQ ID NO: 44, an FIP primer consisting of the nucleotide sequence set forth in SEQ ID NO: 45, and a BIP primer consisting of the nucleotide sequence set forth in SEQ ID NO: 46.
[6] The method according to [5],
   wherein the primer set of (a1) further comprises an LF primer consisting of the nucleotide sequence set forth in SEQ ID NO: 9 and an LB primer consisting of the nucleotide sequence set forth in SEQ ID NO: 10,
   wherein the primer set of (a2) further comprises an LF primer consisting of the nucleotide sequence set forth in SEQ ID NO: 35 and an LB primer consisting of the nucleotide sequence set forth in SEQ ID NO: 36,
   wherein the primer set of (a3) further comprises an LF primer consisting of the nucleotide sequence set forth in SEQ ID NO: 41 and an LB primer consisting of the nucleotide sequence set forth in SEQ ID NO: 42, and
   wherein the primer set of (a4) further comprises an LF primer consisting of the nucleotide sequence set forth in SEQ ID NO: 47 and an LB primer consisting of the nucleotide sequence set forth in SEQ ID NO: 48.
[7] The method according to [4],
   wherein the nucleic acid amplification reaction is a polymerase chain reaction, and
   wherein the primer set is (a5) a primer set comprising a forward primer consisting of the nucleotide sequence set forth in SEQ ID NO: 67 and a reverse primer consisting of the nucleotide sequence set forth in SEQ ID NO: 68.
[8] The method according to any one of [1] to [7], wherein, in the step of performing a nucleic acid amplification reaction, a primer set specific to a dicarboxylate/amino acid:cation symporter gene of *Fusobacterium nucleatum subsp. nucleatum* is further used in conjunction with the primer set specific to a citrate lyase beta subunit gene of *Fusobacterium nucleatum.*
[9] A primer set specific to a citrate lyase beta subunit gene of *Fusobacterium nucleatum* for detecting *Fusobacterium nucleatum.*
[10] The primer set according to [9], wherein the citrate lyase beta subunit gene consists of the nucleotide sequence set forth in any one of SEQ ID NOs: 1 to 4 or a nucleotide sequence having 90% or more sequence identity to the nucleotide sequence set forth in any one of SEQ ID NOs: 1 to 4.
[11] The primer set according to [10], which is specific to
   a region from a 141st to a 415th base,
   a region from a 4th to a 253rd base,
   a region from a 238th to a 561st base,
   a region from a 577th to a 806th base, or
   a region from a 32nd to a 636th base in the nucleotide sequence set forth in any one of SEQ ID NOs: 1 to 4,
   or a region having 90% or more sequence identity to any of these regions.
[12] The primer set according to any one of [9] to [11], which is for a loop-mediated isothermal amplification reaction or a polymerase chain reaction.
[13] The primer set according to [12], which is for a loop-mediated isothermal amplification reaction and is
   (a1) a primer set comprising an F3 primer consisting of the nucleotide sequence set forth in SEQ ID NO: 5, a B3 primer consisting of the nucleotide sequence set forth in SEQ ID NO: 6, an FIP primer consisting of the nucleotide sequence set forth in SEQ ID NO: 7, and a BIP primer consisting of the nucleotide sequence set forth in SEQ ID NO: 8,
   (a2) a primer set comprising an F3 primer consisting of the nucleotide sequence set forth in SEQ ID NO: 31, a B3 primer consisting of the nucleotide sequence set forth in SEQ ID NO: 32, an FIP primer consisting of the nucleotide sequence set forth in SEQ ID NO: 33, and a BIP primer consisting of the nucleotide sequence set forth in SEQ ID NO: 34,
   (a3) a primer set comprising an F3 primer consisting of the nucleotide sequence set forth in SEQ ID NO: 37, a B3 primer consisting of the nucleotide sequence set forth in SEQ ID NO: 38, an FIP primer consisting of the nucleotide sequence set forth in SEQ ID NO: 39, and a BIP primer consisting of the nucleotide sequence set forth in SEQ ID NO: 40, or
   (a4) a primer set comprising an F3 primer consisting of the nucleotide sequence set forth in SEQ ID NO: 43, a B3 primer consisting of the nucleotide sequence set forth in SEQ ID NO: 44, an FIP primer consisting of the nucleotide sequence set forth in SEQ ID NO: 45, and a BIP primer consisting of the nucleotide sequence set forth in SEQ ID NO: 46.
[14] The primer set according to [13],
   wherein the primer set of (a1) further comprises an LF primer consisting of the nucleotide sequence set forth in SEQ ID NO: 9 and an LB primer consisting of the nucleotide sequence set forth in SEQ ID NO: 10,
   wherein the primer set of (a2) further comprises an LF primer consisting of the nucleotide sequence set forth in SEQ ID NO: 35 and an LB primer consisting of the nucleotide sequence set forth in SEQ ID NO: 36,
   wherein the primer set of (a3) further comprises an LF primer consisting of the nucleotide sequence set forth in SEQ ID NO: 41 and an LB primer consisting of the nucleotide sequence set forth in SEQ ID NO: 42, and
   wherein the primer set of (a4) further comprises an LF primer consisting of the nucleotide sequence set forth in SEQ ID NO: 47 and an LB primer consisting of the nucleotide sequence set forth in SEQ ID NO: 48.
[15] The primer set according to [12], which is for a polymerase chain reaction and is (a5) a primer set comprising a forward primer consisting of the nucleotide sequence set forth in SEQ ID NO: 67 and a reverse primer consisting of the nucleotide sequence set forth in SEQ ID NO: 68.
[16] A method for detecting *Fusobacterium nucleatum subsp. nucleatum* in a sample, comprising:
   a step of performing a nucleic acid amplification reaction on a DNA derived from the sample using a primer set specific to a dicarboxylate/amino acid:cation symporter gene of *Fusobacterium nucleatum subsp. nucleatum.*
[17] The method according to [16], wherein the dicarboxylate/amino acid:cation symporter gene consists of the nucleotide sequence set forth in SEQ ID NO: 11 or a nucleotide sequence having 90% or more sequence identity to the nucleotide sequence set forth in SEQ ID NO: 11.
[18] The method according to [17], wherein the primer set is a primer set specific to
   a region from a 860th to a 1121st base,
   a region from a 196th to a 449th base,
   a region from a 580th to a 869th base, or
   a region from a 761st to a 1038th base in the nucleotide sequence set forth in SEQ ID NO: 11,
   or a region having 90% or more sequence identity to any of these regions.
[19] The method according to any one of [16] to [18], wherein the nucleic acid amplification reaction is a loop-mediated isothermal amplification reaction.
[20] The method according to [19], wherein the primer set is
   (b1) a primer set comprising an F3 primer consisting of the nucleotide sequence set forth in SEQ ID NO: 12, a B3 primer consisting of the nucleotide sequence set forth in SEQ ID NO: 13, an FIP primer consisting of the nucleotide sequence set forth in SEQ ID NO: 14, and a BIP primer consisting of the nucleotide sequence set forth in SEQ ID NO: 15,
   (b2) a primer set comprising an F3 primer consisting of the nucleotide sequence set forth in SEQ ID NO: 49, a B3 primer consisting of the nucleotide sequence set forth in SEQ ID NO: 50, an FIP primer consisting of the nucleotide sequence set forth in SEQ ID NO: 51, and a BIP primer consisting of the nucleotide sequence set forth in SEQ ID NO: 52,
   (b3) a primer set comprising an F3 primer consisting of the nucleotide sequence set forth in SEQ ID NO: 55, a B3 primer consisting of the nucleotide sequence set forth in SEQ ID NO: 56, an FIP primer consisting of the nucleotide sequence set forth in SEQ ID NO: 57, and a BIP primer consisting of the nucleotide sequence set forth in SEQ ID NO: 58, or
   (b4) a primer set comprising an F3 primer consisting of the nucleotide sequence set forth in SEQ ID NO: 61, a B3 primer consisting of the nucleotide sequence set forth in SEQ ID NO: 62, an FIP primer consisting of the nucleotide sequence set forth in SEQ ID NO: 63, and a BIP primer consisting of the nucleotide sequence set forth in SEQ ID NO: 64.
[21] The method according to [20],
   wherein the primer set of (b1) further comprises an LF primer consisting of the nucleotide sequence set forth in SEQ ID NO: 16 and an LB primer consisting of the nucleotide sequence set forth in SEQ ID NO: 17,
   wherein the primer set of (b2) further comprises an LF primer consisting of the nucleotide sequence set forth in SEQ ID NO: 53 and an LB primer consisting of the nucleotide sequence set forth in SEQ ID NO: 54,
   wherein the primer set of (b3) further comprises an LF primer consisting of the nucleotide sequence set forth in SEQ ID NO: 59 and an LB primer consisting of the nucleotide sequence set forth in SEQ ID NO: 60, and
   wherein the primer set of (b4) further comprises an LF primer consisting of the nucleotide sequence set forth in SEQ ID NO: 65 and an LB primer consisting of the nucleotide sequence set forth in SEQ ID NO: 66.
[22] A primer set specific to a dicarboxylate/amino acid:cation symporter gene of *Fusobacterium nucleatum subsp. nucleatum* for detecting *Fusobacterium nucleatum subsp. nucleatum.*
[23] The primer set according to [22], wherein the dicarboxylate/amino acid:cation symporter gene consists of the nucleotide sequence set forth in SEQ ID NO: 11 or a nucleotide sequence having 90% or more sequence identity to the nucleotide sequence set forth in SEQ ID NO: 11.
[24] The primer set according to [23], which is specific to
   a region from a 860th to a 1121st base,
   a region from a 196th to a 449th base,
   a region from a 580th to a 869th base, or
   a region from a 761st to a 1038th base in the nucleotide sequence set forth in SEQ ID NO: 11,
   or a region having 90% or more sequence identity to any of these regions.
[25] The primer set according to any one of [22] to [24], which is a primer set for a loop-mediated isothermal amplification reaction.
[26] The primer set according to [25], which is
   (b1) a primer set comprising an F3 primer consisting of the nucleotide sequence set forth in SEQ ID NO: 12, a B3 primer consisting of the nucleotide sequence set forth in SEQ ID NO: 13, an FIP primer consisting of the nucleotide sequence set forth in SEQ ID NO: 14, and a BIP primer consisting of the nucleotide sequence set forth in SEQ ID NO: 15,
   (b2) a primer set comprising an F3 primer consisting of the nucleotide sequence set forth in SEQ ID NO: 49, a B3 primer consisting of the nucleotide sequence set forth in SEQ ID NO: 50, an FIP primer consisting of the nucleotide sequence set forth in SEQ ID NO: 51, and a BIP primer consisting of the nucleotide sequence set forth in SEQ ID NO: 52,
   (b3) a primer set comprising an F3 primer consisting of the nucleotide sequence set forth in SEQ ID NO: 55, a B3 primer consisting of the nucleotide sequence set forth in SEQ ID NO: 56, an FIP primer consisting of the nucleotide sequence set forth in SEQ ID NO: 57, and a BIP primer consisting of the nucleotide sequence set forth in SEQ ID NO: 58, or
   (b4) a primer set comprising an F3 primer consisting of the nucleotide sequence set forth in SEQ ID NO: 61, a B3 primer consisting of the nucleotide sequence set forth in SEQ ID NO: 62, an FIP primer consisting of the nucleotide sequence set forth in SEQ ID NO: 63, and a BIP primer consisting of the nucleotide sequence set forth in SEQ ID NO: 64.
[27] The primer set according to [26],
   wherein the primer set of (b1) further comprises an LF primer consisting of the nucleotide sequence set forth in SEQ ID NO: 16 and an LB primer consisting of the nucleotide sequence set forth in SEQ ID NO: 17,
   wherein the primer set of (b2) further comprises an LF primer consisting of the nucleotide sequence set forth in SEQ ID NO: 53 and an LB primer consisting of the nucleotide sequence set forth in SEQ ID NO: 54,
   wherein the primer set of (b3) further comprises an LF primer consisting of the nucleotide sequence set forth in SEQ ID NO: 59 and an LB primer consisting of the nucleotide sequence set forth in SEQ ID NO: 60, and
   wherein the primer set of (b4) further comprises an LF primer consisting of the nucleotide sequence set forth in SEQ ID NO: 65 and an LB primer consisting of the nucleotide sequence set forth in SEQ ID NO: 66.

### Advantageous Effects of Invention

According to the method according to one aspect of the present invention, *F*. *nucleatum* can be specifically detected. In addition, according to the method according to another aspect of the present invention, *F*. *nucleatum subsp. nucleatum* can be specifically detected. The present invention is particularly useful in that it enables specific detection of four subspecies of *F*. *nucleatum* or *F*. *nucleatum subsp. nucleatum* contained in clinical specimens such as saliva, feces, and tissues without undergoing culture.

### Description of Embodiment

A method for detecting *Fusobacterium nucleatum* (*F. nucleatum)* in a sample according to one aspect of the present invention includes: a step of performing a nucleic acid amplification reaction on a DNA derived from the sample using a primer set specific to a citrate lyase beta subunit gene (CitE) of *F*. *nucleatum* (hereinafter referred to as a nucleic acid amplification step).

The sample is not particularly limited and may be, for example, a sample collected from a subject suspected of being infected with *F*. *nucleatum.* The sample may be, for example, a biological sample such as nasal mucus, nasal suction fluid, a nasal swab, a pharyngeal swab, vaginal swab fluid, saliva, blood, plasma, serum, menstrual blood, urine, feces, tissues, or cells. The sample may be a sample obtained by subjecting the above-described biological sample to treatment such as separation, concentration, and purification.

The DNA derived from the sample may be a DNA isolated from the sample, or a DNA not isolated from the sample and dissolved in the sample. Therefore, the method for detecting *F*. *nucleatum* in a sample according to the present aspect may include a step of extracting a DNA from the sample and/or a step of isolating a DNA from the sample, before the nucleic acid amplification step. The extraction and isolation of the DNA may be performed by known techniques.

In the present specification, "citrate lyase beta subunit gene" refers to a gene encoding a beta subunit of citrate lyase. The beta subunit of citrate lyase may also be referred to by other names such as "citrate lyase subunit beta," "citrate (pro-3S)-lyase subunit beta," "citrate (pro-3S)-lyase, beta subunit," and "citrate lyase beta chain", and genes encoding these proteins are also within the scope of the term "citrate lyase beta subunit gene", as long as these proteins function as beta subunits of citrate lyase.

The citrate lyase beta subunit gene of *F*. *nucleatum* may be, for example, a gene consisting of the nucleotide sequence set forth in any one of SEQ ID NOs: 1 to 4 or a nucleotide sequence having 90% or more sequence identity to the nucleotide sequence set forth in any one of SEQ ID NOs: 1 to 4. SEQ ID NOs: 1 to 4 respectively show nucleotide sequences of the citrate lyase beta subunit gene of *F*. *nucleatum* shown in the following table.

**[Table 1]**

| SEQ ID NO | Subspecies | Strain | Accession |
|---|---|---|---|
| 1 | *F. nucleatum subsp. nucleatum* | ATCC25586 | CP028101 |
| 2 | *F. nucleatum subsp. animalis* | ATCC51191 | CP012713 |
| 3 | *F. nucleatum subsp. polymorphum* | ATCC10953 | LN831027 |
| 4 | *F. nucleatum subsp. vincentii* | ATCC49256 | CP024749 |

From the viewpoint of more specifically detecting *F*. *nucleatum,* the nucleotide sequence having 90% or more sequence identity to the nucleotide sequence set forth in any one of SEQ ID NOs: 1 to 4 is preferably a nucleotide sequence having 92% or more, 93% or more, or 95% or more sequence identity to the nucleotide sequence set forth in any one of SEQ ID NOs: 1 to 4.

In the present specification, a primer set specific to a certain gene or a certain region in a gene refers to a combination of one or more forward primers and one or more reverse primers (also referred to as backward primers) which anneals to (that is, specifically bound to) a nucleotide sequence of the gene or the region (that is, a target gene or a target region) or a nucleotide sequence complementary thereto. By performing a nucleic acid amplification reaction using a primer set specific to a target gene or a target region, a part or the entire length of the target gene or region is amplified.

Because the citrate lyase beta subunit gene of *F*. *nucleatum* has high sequence identity among subspecies, a primer set specific to a citrate lyase beta subunit gene of one subspecies is also specific to citrate lyase beta subunit genes of all other subspecies. Namely, a primer set specific to a citrate lyase beta subunit gene of *F*. *nucleatum* is a primer set specific to citrate lyase beta subunit genes of all subspecies of *F*. *nucleatum.* Namely, for example, it can be said that a primer set specific to a citrate lyase beta subunit gene consisting of the nucleotide sequence set forth in any one of SEQ ID NOs: 1 to 4 is naturally a primer set specific to all of a citrate lyase beta subunit gene consisting of the nucleotide sequence set forth in SEQ ID NO: 1, a citrate lyase beta subunit gene consisting of the nucleotide sequence set forth in SEQ ID NO: 2, a citrate lyase beta subunit gene consisting of the nucleotide sequence set forth in SEQ ID NO: 3, and a citrate lyase beta subunit gene consisting of the nucleotide sequence set forth in SEQ ID NO: 4. In addition, such a primer set can be said to be a primer set also specific to a citrate lyase beta subunit gene consisting of a nucleotide sequence having 90% or more, 92% or more, 93% or more, or 95% or more sequence identity to the nucleotide sequence set forth in SEQ ID NO: 1, the nucleotide sequence set forth in SEQ ID NO: 2, the nucleotide sequence set forth in SEQ ID NO: 3, and the nucleotide sequence set forth in SEQ ID NO: 4.

The primer set can be designed based on an arbitrary region in the citrate lyase beta subunit gene. The primer set may be designed based on, for example,
(i) a region from the 141st to the 415th base,
(ii) a region from the 4th to the 253rd base,
(iii) a region from the 238th to the 561st base,
(iv) a region from the 577th to the 806th base, or
(v) a region from the 32nd to the 636th base
   in the citrate lyase beta subunit gene. Namely, the primer set specific to a citrate lyase beta subunit gene may be, for example, a primer set specific to
      (i) a region from the 141st to the 415th base,
      (ii) a region from the 4th to the 253rd base,
      (iii) a region from the 238th to the 561st base,
      (iv) a region from the 577th to the 806th base, or
      (v) a region from the 32nd to the 636th base
   in the nucleotide sequence set forth in any one of SEQ ID NOs: 1 to 4. Alternatively, the primer set specific to a citrate lyase beta subunit gene may be a primer set specific to a region corresponding to any of the regions (i) to (v) above in the nucleotide sequence having 90% or more sequence identity to the nucleotide sequence set forth in any one of SEQ ID NOs: 1 to 4, and namely, a primer set specific to a region having 90% or more, 92% or more, 93% or more, or 95% or more sequence identity to any of the regions (i) to (v) above. As described above, the primer set specific to the region from the 141st to the 415th base in the nucleotide sequence set forth in any one of SEQ ID NOs: 1 to 4 can be said to be a primer set specific to the regions from the 141st to the 415th base in each of the nucleotide sequences set forth in SEQ ID NOs: 1 to 4, and such a primer set can be said to be a primer set specific to a citrate lyase beta subunit gene consisting of a region having 90% or more, 92% or more, 93% or more, or 95% or more sequence identity to those regions. The same applies to primer sets specific to the other regions.

The primer set may be appropriately designed by those skilled in the art based on the disclosure of known literature, depending on the type of nucleic acid amplification reaction. In addition, the number (type) of the forward primers and the reverse primers constituting the primer set may also be appropriately determined by those skilled in the art, depending on the type of nucleic acid amplification reaction.

The nucleic acid amplification reaction may be, for example, a polymerase chain reaction (PCR), a loop-mediated isothermal amplification (LAMP) reaction, a transcription-mediated amplification (TMA) reaction, or a nucleic acid sequence-based amplification (NASBA) reaction.

If the nucleic acid amplification reaction is a LAMP reaction, the primer set contains an F3 primer, an FIP primer, and optionally an LF primer as forward primers and contains a B3 primer, a BIP primer, and optionally an LB primer as reverse primers. The method for designing these primers based on a certain target gene is known.

Examples of the primer set for a LAMP reaction include the following primer sets of (a1) to (a4). The position of each base is the position in the nucleotide sequence set forth in any one of SEQ ID NOs: 1 to 4.
(a1) A primer set containing:
   an F3 primer designed based on a region (region F3) from the 141st to the 163rd base;
   a B3 primer designed based on a region (region B3) complementary to a region from the 394th to the 415th base;
   an FIP primer of which the 5' end is designed based on a region (region F1c) complementary to a region from the 268th to the 292nd base and the 3' end is designed based on a region (region F2) from the 179th to the 205th base; and
   a BIP primer of which the 5' end is designed based on a region (region B1c) from the 301st to the 325th base and the 3' end is designed based on a region (region B2) complementary to a region from the 367th to the 386th base, and
   optionally containing:
   an LF primer designed based on a region complementary to a region from the 226th to the 253rd base; and
   an LB primer designed based on a region from the 336th to the 366th base.
(a2) A primer set containing:
   an F3 primer designed based on a region (region F3) from the 4th to the 29th base;
   a B3 primer designed based on a region (region B3) complementary to a region from the 231st to the 253rd base;
   an FIP primer of which the 5' end is designed based on a region (region F1c) complementary to a region from the 106th to the 131st base and the 3' end is designed based on a region (region F2) from the 40th to the 63rd base; and
   a BIP primer of which the 5' end is designed based on a region (region B1c) from the 133rd to the 163rd base and the 3' end is designed based on a region (region B2) complementary to a region from the 200th to the 223rd base, and
   optionally containing:
   an LF primer designed based on a region complementary to a region from the 64th to the 93rd base; and
   an LB primer designed based on a region from the 166th to the 199th base.
(a3) A primer set containing:
   an F3 primer designed based on a region (region F3) from the 238th to the 259th base;
   a B3 primer designed based on a region (region B3) complementary to a region from the 544th to the 561st base;
   an FIP primer of which the 5' end is designed based on a region (region F1c) complementary to a region from the 428th to the 454th base and the 3' end is designed based on a region (region F2) from the 269th to the 295th base; and
   a BIP primer of which the 5' end is designed based on a region (region B1c) from the 465th to the 490th base and the 3' end is designed based on a region (region B2) complementary to a region from the 514th to the 536th base, and
   optionally containing:
   an LF primer designed based on a region complementary to a region from the 398th to the 427th base; and
   an LB primer designed based on a region from the 486th to the 513th base.
(a4) A primer set containing:
   an F3 primer designed based on a region (region F3) from the 577th to the 599th base;
   a B3 primer designed based on a region (region B3) complementary to a region from the 784th to the 806th base;
   an FIP primer of which the 5' end is designed based on a region (region F1c) complementary to a region from the 652nd to the 677th base and the 3' end is designed based on a region (region F2) from the 601st to the 625th base; and
   a BIP primer of which the 5' end is designed based on a region (region B1c) from the 689th to the 718th base and the 3' end is designed based on a region (region B2) complementary to a region from the 766th to the 783rd base, and
   optionally containing:
   an LF primer designed based on a region complementary to a region from the 626th to the 658th base; and
   an LB primer designed based on a region from the 722nd to the 756th base.

In the present specification, "primer designed based on a certain region" means a primer designed to anneal to a complementary sequence of the region, and usually has the same nucleotide sequence as that of the region or has a nucleotide sequence having 90% or more, 91% or more, 92% or more, 93% or more, or 94% or more, 95% or more, 96% or more, 97% or more, 98% or more, or 99% or more sequence identity to the nucleotide sequence.

Specific examples of the above-described primer set of (a1) include a primer set containing an F3 primer consisting of the nucleotide sequence set forth in SEQ ID NO: 5, a B3 primer consisting of the nucleotide sequence set forth in SEQ ID NO: 6, an FIP primer consisting of the nucleotide sequence set forth in SEQ ID NO: 7, and a BIP primer consisting of the nucleotide sequence set forth in SEQ ID NO: 8, and optionally an LF primer consisting of the nucleotide sequence set forth in SEQ ID NO: 9 and an LB primer consisting of the nucleotide sequence set forth in SEQ ID NO: 10. In addition, the F3, B3, FIP, BIP, LF, and LB primers may consist of nucleotide sequences having 90% or more sequence identity to the nucleotide sequences set forth in SEQ ID NOs: 5, 6, 7, 8, 9, and 10, respectively.

Specific examples of the above-described primer set of (a2) include a primer set containing an F3 primer consisting of the nucleotide sequence set forth in SEQ ID NO: 31, a B3 primer consisting of the nucleotide sequence set forth in SEQ ID NO: 32, an FIP primer consisting of the nucleotide sequence set forth in SEQ ID NO: 33, and a BIP primer consisting of the nucleotide sequence set forth in SEQ ID NO: 34, and optionally an LF primer consisting of the nucleotide sequence set forth in SEQ ID NO: 35 and an LB primer consisting of the nucleotide sequence set forth in SEQ ID NO: 36. In addition, the F3, B3, FIP, BIP, LF, and LB primers may consist of nucleotide sequences having 90% or more sequence identity to the nucleotide sequences set forth in SEQ ID NOs: 31, 32, 33, 34, 35, and 36, respectively.

Specific examples of the above-described primer set of (a3) include a primer set containing an F3 primer consisting of the nucleotide sequence set forth in SEQ ID NO: 37, a B3 primer consisting of the nucleotide sequence set forth in SEQ ID NO: 38, an FIP primer consisting of the nucleotide sequence set forth in SEQ ID NO: 39, and a BIP primer consisting of the nucleotide sequence set forth in SEQ ID NO: 40, and optionally an LF primer consisting of the nucleotide sequence set forth in SEQ ID NO: 41 and an LB primer consisting of the nucleotide sequence set forth in SEQ ID NO: 42. In addition, the F3, B3, FIP, BIP, LF, and LB primers may consist of nucleotide sequences having 90% or more sequence identity to the nucleotide sequences set forth in SEQ ID NOs: 37, 38, 39, 40, 41, and 42, respectively.

Specific examples of the above-described primer set of (a4) include a primer set containing an F3 primer consisting of the nucleotide sequence set forth in SEQ ID NO: 43, a B3 primer consisting of the nucleotide sequence set forth in SEQ ID NO: 44, an FIP primer consisting of the nucleotide sequence set forth in SEQ ID NO: 45, and a BIP primer consisting of the nucleotide sequence set forth in SEQ ID NO: 46, and optionally an LF primer consisting of the nucleotide sequence set forth in SEQ ID NO: 47 and an LB primer consisting of the nucleotide sequence set forth in SEQ ID NO: 48. In addition, the F3, B3, FIP, BIP, LF, and LB primers may consist of nucleotide sequences having 90% or more sequence identity to the nucleotide sequences set forth in SEQ ID NOs: 43, 44, 45, 46, 47, and 48, respectively.

The above-described sequence identities may be 91% or more, 92% or more, 93% or more, 94% or more, 95% or more, 96% or more, 97% or more, 98% or more, or 99% or more.

If the nucleic acid amplification reaction is PCR, examples of the primer set for PCR include the following primer set of (a5). The position of each base is the position in the nucleotide sequence set forth in any one of SEQ ID NOs: 1 to 4.
(a5)
A primer set containing:
a forward primer designed based on a region from the 32nd to the 59th base; and
a reverse primer designed based on a region complementary to a region from the 613th to the 636th base.

Specific examples of the above-described primer set of (a5) include a primer set containing a forward primer consisting of the nucleotide sequence set forth in SEQ ID NO: 67 and a reverse primer consisting of the nucleotide sequence set forth in SEQ ID NO: 68. In addition, the forward primer and the reverse primer may consist of nucleotide sequences having 90% or more sequence identity to the nucleotide sequences set forth in SEQ ID NOs: 67 and 68, respectively.

The above-described sequence identity may be 91% or more, 92% or more, 93% or more, 94% or more, 95% or more, 96% or more, 97% or more, 98% or more, or 99% or more.

In the above-described nucleic acid amplification step, a primer set specific to a dicarboxylate/amino acid:cation symporter gene of *F*. *nucleatum subsp. nucleatum* may be further used together with the primer set specific to a citrate lyase beta subunit gene of *F*. *nucleatum.* Combined use with the primer set specific to a dicarboxylate/amino acid:cation symporter gene of *F*. *nucleatum subsp. nucleatum* can improve the detection sensitivity of *F*. *nucleatum subsp. nucleatum.*

In the above-described nucleic acid amplification step, known reagents, such as a DNA polymerase and deoxynucleoside triphosphates (dNTPs: dATP, dTTP, dCTP, and dGTP), for performing a nucleic acid amplification reaction may be further used. Examples of other known reagents for performing a nucleic acid amplification reaction include, for example: a buffer solution or a salt that provides suitable conditions for enzymatic reactions; and a protective agent, such as dithiothreitol, for stabilizing an enzyme or the DNA derived from the sample.

The DNA polymerase may be selected as appropriate, depending on the type of nucleic acid amplification reaction, and may be, for example, Bst DNA polymerase (large fragment), Bca(exo-) DNA polymerase, Csa DNA polymerase, Klenow fragment of Escherichia coli DNA polymerase I, or Taq DNA polymerase.

In the above-described nucleic acid amplification step, a labeling probe or fluorescent intercalator for detecting an amplification product of the nucleic acid amplification reaction may be further used. As the labeling probe, a known fluorescence labeling probe such as a fluorescence quenching probe (Quenching Probe: QProbe (registered trademark)) may be used, for example. As the fluorescent intercalator, a known fluorescent intercalator such as SYTO (trademark) 63 Red Fluorescent Nucleic Acid Stain or SYBR (trademark) Green I Nucleic Acid Gel Stain (both manufactured by Thermo Fisher Scientific Inc.) may be used, for example.

The above-described nucleic acid amplification step may be performed, for example, by incubating a reaction solution containing the DNA derived from the sample and the above-described primer sets. The reaction solution may further contain the above-described known reagents for performing a nucleic acid amplification reaction and the above-described labeling probe or fluorescent intercalator. The temperature and time of incubation (that is, the temperature and time of the reaction) may be set as appropriate, depending on the type of the nucleic acid amplification reaction, the type of the DNA polymerase, and the melting temperature of the primers. For example, in the case of the LAMP reaction, the reaction temperature is not particularly limited as long as it is a temperature at which each primer can be annealed to the citrate lyase beta subunit gene, and may be, for example, 65°C or lower or 60°C or lower, and the reaction time may be, for example, 90 minutes or shorter or 60 minutes or shorter.

The concentration of each primer in the reaction solution may be adjusted as appropriate, depending on the type of the nucleic acid amplification reaction. For example, in the case of the LAMP reaction, the concentration of the FIP primer and the concentration of the BIP primer in the reaction solution may be, for example, 0.8 to 2.4 µM, the concentration of an F3 primer and the concentration of a B3 primer may be, for example, 0.1 to 0.3 µM, and the concentration of an LF primer and the concentration of an LB primer may be, for example, 0.4 to 1.2 µM.

The method for detecting *F*. *nucleatum* in a sample according to the present aspect of the present invention may further include a step of detecting an amplification product of the nucleic acid amplification reaction. If the presence of the amplification product is detected, it can be determined that *F*. *nucleatum* is present in the sample. On the other hand, if the presence of the amplification product is not detected, it can be determined that *F*. *nucleatum* is not present in the sample. The detection of the amplification product may be performed after the nucleic acid amplification step or may be performed in real-time during the nucleic acid amplification step.

The method for detecting the amplification product is not particularly limited, and known technology may be used. The amplification product may be detected, for example, by using the above-described labeling probe or fluorescent intercalator, or by performing electrophoresis on the reaction solution. Alternatively, if magnesium ions are contained in the reaction solution, the amplification product may be detected by measuring the turbidity of the reaction solution. Magnesium ions react with pyrophosphate ions, which are by-products of nucleic acid synthesis, to produce white magnesium pyrophosphate. From the viewpoint of more easily detecting *F*. *nucleatum* in a sample, the detection of the amplification product is preferably performed by using the labeling probe or the fluorescent intercalator or by measuring the turbidity of the reaction solution.

According to the method for detecting *F*. *nucleatum* in a sample according to the present aspect of the present invention, *F*. *nucleatum* can be specifically detected by using a primer set specific to a citrate lyase beta subunit gene, the gene having high sequence identity among *F*. *nucleatum* subspecies while having low sequence identity to other species of *Fusobacterium.* In addition, since a citrate lyase beta subunit gene has high sequence identity among *F*. *nucleatum* subspecies, four subspecies of *F*. *nucleatum* can be detected with a single primer set specific to the citrate lyase beta subunit gene, without preparing a primer set for each subspecies. The existence of a gene with such a species-specific sequence is extremely rare, and it can be said that it could not have been discovered without the ingenuity and excellent information processing techniques of the present inventor and the considerable amount of time spent.

Specifically, when designing primers in general, a region of about several hundred bp that can serve as an amplification region is searched, using homology and cross-reactivity suitable for the purpose as indicators. When the present inventor searched for such a region in the genome of *F*. *nucleatum,* it was found that even strains belonging to the same subspecies had diverse sequences. Therefore, to conduct a more comprehensive search, it was necessary to refer to the public genome sequences of the American Type Culture Collection (ATCC) and multiple databases such as the NCBI. However, it was difficult to find a region of several hundred bp with high homology among *F*. *nucleatum* subspecies and low homology to other species of *Fusobacterium.* Therefore, the present inventor deviated from the general method, and subdivided the genome sequences of *Fusobacterium* into about 20 bp to search for common sequences among *F*. *nucleatum* subspecies. Comprehensive analysis of cross-reactivity of more than 30,000 common sequences revealed a rare region, the citrate lyase beta subunit gene, specific solely to *F*. *nucleatum.*

As described above, *F*. *nucleatum* can be specifically detected by using the above-described primer set specific to a citrate lyase beta subunit gene of *F*. *nucleatum.* Therefore, another aspect of the present invention can be said to be the above-described primer set for detecting *F*. *nucleatum,* which is specific to the citrate lyase beta subunit gene of *F*. *nucleatum.*

In addition, another aspect of the present invention is a kit for detecting *F*. *nucleatum,* containing the above-described primer set specific to a citrate lyase beta subunit gene of *F*. *nucleatum.* The kit may further contain the above-described known reagents for performing a nucleic acid amplification reaction, such as a DNA polymerase, dNTPs, a buffer solution, a salt, and a protective agent, and may further contain the above-described labeling probe or fluorescent intercalator. In addition, the kit may further contain a primer set specific to a dicarboxylate/amino acid:cation symporter gene of *F*. *nucleatum subsp. nucleatum* described below.

A method for detecting *F*. *nucleatum subsp. nucleatum* in a sample according to one aspect of the present invention includes a step of performing a nucleic acid amplification reaction on a DNA derived from the sample using a primer set specific to a dicarboxylate/amino acid:cation symporter (DAACS) gene of *F*. *nucleatum subsp. nucleatum* (hereinafter referred to as a nucleic acid amplification step).

The sample is not particularly limited and may be, for example, a sample collected from a subject suspected of being infected with *F*. *nucleatum subsp. nucleatum.* Examples of the sample include the same samples as those exemplified in the method for detecting *F*. *nucleatum* in a sample according to the above-described aspect.

Details regarding the DNA derived from the sample are as described in the method for detecting *F*. *nucleatum* in a sample according to the above-described aspect.

In the present specification, "dicarboxylate/amino acid:cation symporter gene" refers to a gene encoding a dicarboxylate/amino acid:cation symporter. The dicarboxylate/amino acid:cation symporter may also be referred to by other names such as "proton/sodium-aspartate symport protein", "glutamate:protein symporter," and "C4-dicarboxylate transport protein", and genes encoding these proteins are also within the scope of the term "dicarboxylate/amino acid:cation symporter gene", as long as these proteins function as dicarboxylate/amino acid:cation symporters (that is, as long as these proteins have a dicarboxylate/amino acid:cation symport activity).

The DAACS gene of *F*. *nucleatum subsp. nucleatum* may be, for example, a gene consisting of the nucleotide sequence set forth in SEQ ID NO: 11 or a nucleotide sequence having 90% or more sequence identity to the nucleotide sequence set forth in SEQ ID NO: 11. SEQ ID NO: 11 shows a nucleotide sequence of the DAACS gene of the strain ATCC 25586 (accession: CP028101) of *F*. *nucleatum subsp. nucleatum.* The nucleotide sequence having 90% or more sequence identity to the nucleotide sequence set forth in SEQ ID NO: 11 is preferably a nucleotide sequence having 95% or more, 99% or more, or 99.5% or more sequence identity to the nucleotide sequence set forth in SEQ ID NO: 11.

The primer set can be designed based on an arbitrary region in the DAACS gene. The primer set may be designed based on, for example,
(I) a region from the 860th to the 1121st base,
(II) a region from the 196th to the 449th base,
(III) a region from the 580th to the 869th base, or
(IV) a region from the 761st to the 1038th base
   in the DAACS gene. Namely, the primer set specific to a DAACS gene may be, for example, a primer set specific to
   (I) a region from the 860th to the 1121st base,
   (II) a region from the 196th to the 449th base,
   (III) a region from the 580th to the 869th base, or
   (IV) a region from the 761st to the 1038th base in the nucleotide sequence set forth in SEQ ID NO: 11. Alternatively, the primer set specific to a DAACS gene may be a primer set specific to a region corresponding to any of the regions of (I) to (IV) above in the nucleotide sequence having 90% or more sequence identity to the nucleotide sequence set forth in SEQ ID NO: 11, and namely, a primer set specific to a region having 90% or more, 95% or more, 99% or more, or 99.5% or more sequence identity to any of the regions of (I) to (IV) above.

The primer set may be appropriately designed by those skilled in the art based on the disclosure of known literature, depending on the type of nucleic acid amplification reaction. In addition, the number (type) of the forward primers and the reverse primers constituting the primer set may also be appropriately determined by those skilled in the art, depending on the type of nucleic acid amplification reaction.

The nucleic acid amplification reaction may be, for example, a PCR, a LAMP reaction, a TMA reaction, or a NASBA reaction.

If the nucleic acid amplification reaction is a LAMP reaction, the primer set contains an F3 primer, an FIP primer, and optionally an LF primer as forward primers and contains a B3 primer, a BIP primer, and optionally an LB primer as reverse primers.

Examples of the primer set for a LAMP reaction include the following primer sets of (b1) to (b4). The position of each base is the position in the nucleotide sequence set forth in SEQ ID NO: 11.
(b1) A primer set containing:
   an F3 primer designed based on a region (region F3) from the 860th to the 885th base;
   a B3 primer designed based on a region (region B3) complementary to a region from the 1098th to the 1121st base;
   an FIP primer of which the 5' end is designed based on a region (region F1c) complementary to a region from the 964th to the 993rd base and the 3' end is designed based on a region (region F2) from the 892nd to the 914th base; and
   a BIP primer of which the 5' end is designed based on a region (region B1c) from the 997th to the 1016th base and the 3' end is designed based on a region (region B2) complementary to a region from the 1070th to the 1092nd base, and
   optionally containing:
   an LF primer designed based on a region complementary to a region from the 882nd to the 925th base; and
   an LB primer designed based on a region from the 1032nd to the 1064th base.
(b2) A primer set containing:
   an F3 primer designed based on a region (region F3) from the 196th to the 220th base;
   a B3 primer designed based on a region (region B3) complementary to a region from the 420th to the 449th base;
   an FIP primer of which the 5' end is designed based on a region (region F1c) complementary to a region from the 285th to the 307th base and the 3' end is designed based on a region (region F2) from the 230th to the 254th base; and
   a BIP primer of which the 5' end is designed based on a region (region B1c) from the 317th to the 342nd base and the 3' end is designed based on a region (region B2) complementary to a region from the 397th to the 417th base, and
   optionally containing:
   an LF primer designed based on a region complementary to a region from the 256th to the 284th base; and
   an LB primer designed based on a region from the 355th to the 380th base.
(b3) A primer set containing:
   an F3 primer designed based on a region (region F3) from the 580th to the 605th base;
   a B3 primer designed based on a region (region B3) complementary to a region from the 844th to the 869th base;
   an FIP primer of which the 5' end is designed based on a region (region F1c) complementary to a region from the 688th to the 717th base and the 3' end is designed based on a region (region F2) from the 619th to the 648th base; and
   a BIP primer of which the 5' end is designed based on a region (region B1c) from the 735th to the 759th base and the 3' end is designed based on a region (region B2) complementary to a region from the 813th to the 834th base, and
   optionally containing:
   an LF primer designed based on a region complementary to a region from the 649th to the 684th base; and
   an LB primer designed based on a region from the 770th to the 792nd base.
(b4) A primer set containing:
   an F3 primer designed based on a region (region F3) from the 761st to the 780th base;
   a B3 primer designed based on a region (region B3) complementary to a region from the 1017th to the 1038th base;
   an FIP primer of which the 5' end is designed based on a region (region F1c) complementary to a region from the 855th to the 884th base and the 3' end is designed based on a region (region F2) from the 785th to the 810th base; and
   a BIP primer of which the 5' end is designed based on a region (region B1c) from the 894th to the 921st base and the 3' end is designed based on a region (region B2) complementary to a region from the 975th to the 996th base, and
   optionally containing:
   an LF primer designed based on a region complementary to a region from the 823rd to the 854th base; and
   an LB primer designed based on a region from the 932nd to the 965th base.

Specific examples of the above-described primer set of (b1) include a primer set containing an F3 primer consisting of the nucleotide sequence set forth in SEQ ID NO: 12, a B3 primer consisting of the nucleotide sequence set forth in SEQ ID NO: 13, an FIP primer consisting of the nucleotide sequence set forth in SEQ ID NO: 14, and a BIP primer consisting of the nucleotide sequence set forth in SEQ ID NO: 15, and optionally an LF primer consisting of the nucleotide sequence set forth in SEQ ID NO: 16 and an LB primer consisting of the nucleotide sequence set forth in SEQ ID NO: 17. In addition, the F3, B3, FIP, BIP, LF, and LB primers may consist of nucleotide sequences having 90% or more sequence identity to the nucleotide sequences set forth in SEQ ID NOs: 12, 13, 14, 15, 16, and 17, respectively.

Specific examples of the above-described primer set of (b2) include a primer set containing an F3 primer consisting of the nucleotide sequence set forth in SEQ ID NO: 49, a B3 primer consisting of the nucleotide sequence set forth in SEQ ID NO: 50, an FIP primer consisting of the nucleotide sequence set forth in SEQ ID NO: 51, and a BIP primer consisting of the nucleotide sequence set forth in SEQ ID NO: 52, and optionally an LF primer consisting of the nucleotide sequence set forth in SEQ ID NO: 53 and an LB primer consisting of the nucleotide sequence set forth in SEQ ID NO: 54. In addition, the F3, B3, FIP, BIP, LF, and LB primers may consist of nucleotide sequences having 90% or more sequence identity to the nucleotide sequences set forth in SEQ ID NOs: 49, 50, 51, 52, 53, and 54, respectively.

Specific examples of the above-described primer set of (b3) include a primer set containing an F3 primer consisting of the nucleotide sequence set forth in SEQ ID NO: 55, a B3 primer consisting of the nucleotide sequence set forth in SEQ ID NO: 56, an FIP primer consisting of the nucleotide sequence set forth in SEQ ID NO: 57, and a BIP primer consisting of the nucleotide sequence set forth in SEQ ID NO: 58, and optionally an LF primer consisting of the nucleotide sequence set forth in SEQ ID NO: 59 and an LB primer consisting of the nucleotide sequence set forth in SEQ ID NO: 60. In addition, the F3, B3, FIP, BIP, LF, and LB primers may consist of nucleotide sequences having 90% or more sequence identity to the nucleotide sequences set forth in SEQ ID NOs: 55, 56, 57, 58, 59, and 60, respectively.

Specific examples of the above-described primer set of (b4) include a primer set containing an F3 primer consisting of the nucleotide sequence set forth in SEQ ID NO: 61, a B3 primer consisting of the nucleotide sequence set forth in SEQ ID NO: 62, an FIP primer consisting of the nucleotide sequence set forth in SEQ ID NO: 63, and a BIP primer consisting of the nucleotide sequence set forth in SEQ ID NO: 64, and optionally an LF primer consisting of the nucleotide sequence set forth in SEQ ID NO: 65 and an LB primer consisting of the nucleotide sequence set forth in SEQ ID NO: 66. In addition, the F3, B3, FIP, BIP, LF, and LB primers may consist of nucleotide sequences having 90% or more sequence identity to the nucleotide sequences set forth in SEQ ID NOs: 61, 62, 63, 64, 65, and 66, respectively.

The above-described sequence identities may be 91% or more, 92% or more, 93% or more, 94% or more, 95% or more, 96% or more, 97% or more, 98% or more, or 99% or more.

In the above-described nucleic acid amplification step, known reagents for performing a nucleic acid amplification reaction and a labeling probe or fluorescent intercalator for detecting an amplification product of the nucleic acid amplification reaction may be further used. Examples of the known reagents for performing a nucleic acid amplification reaction, and the labeling probe and fluorescent intercalator include the same reagents, labeling probe, and fluorescent intercalator as those exemplified in the method for detecting *F*. *nucleatum* in a sample according to the above-described aspect.

The above-described nucleic acid amplification step may be performed, for example, by incubating a reaction solution containing the DNA derived from the sample and the above-described primer sets. The reaction solution may further contain the above-described known reagents for performing a nucleic acid amplification reaction and the above-described labeling probe or fluorescent intercalator. The temperature and time of incubation (that is, the temperature and time of the reaction) and the concentration of each primer in the reaction solution may be the same as those in the method for detecting *F*. *nucleatum* in a sample according to the above-described aspect.

The method for detecting *F*. *nucleatum subsp. nucleatum* in a sample according to the present aspect of the present invention may further include a step of detecting an amplification product of the nucleic acid amplification reaction. If the presence of the amplification product is detected, it can be determined that *F*. *nucleatum subsp. nucleatum* is present in the sample. On the other hand, if the presence of the amplification product is not detected, it can be determined that *F*. *nucleatum subsp. nucleatum* is not present in the sample. The detection of the amplification product may be performed after the nucleic acid amplification step or may be performed in real-time during the nucleic acid amplification step. The details of the method for detecting the amplification product are as described in the method for detecting *F*. *nucleatum* in a sample according to the above-described aspect.

According to the method for detecting *F*. *nucleatum subsp. nucleatum* in a sample according to the present aspect of the present invention, *F*. *nucleatum subsp. nucleatum* can be specifically detected by using a primer set specific to a DAACS gene, the gene having high sequence identity among strains of *F*. *nucleatum subsp. Nucleatum,* while having low sequence identity to other subspecies of *F*. *nucleatum* and other species of *Fusobacterium.* In addition, as described above, although strains belonging to the same *F*. *nucleatum* subspecies have diverse sequences, the DAACS gene has high sequence identity among *F*. *nucleatum subsp. nucleatum* strains, and thus, diverse strains of *F*. *nucleatum subsp. nucleatum* can be detected with a single primer set specific to the DAACS gene, without preparing a primer set for each strain. Furthermore, according to the primer set specific to a DAACS gene, it is possible to achieve higher detection sensitivity compared to using primer sets specific to other regions of the genome of *F*. *nucleatum subsp. nucleatum.* The existence of a gene with such a subspecies-specific sequence is extremely rare, and it can be said that it could not have been discovered without the ingenuity of the present inventor.

The present inventor first used sequence comparison software "Mauve" (Darling, A. C. et al., Genome Res., 2004, 14:1394-1403) to search for sequences specific to *F*. *nucleatum subsp. nucleatum.* As a result, although multiple sequences specific to *F*. *nucleatum subsp. nucleatum* were found, when primer sets specific to those regions were prepared, it was only the DAACS gene for which a primer set with sufficient detection sensitivity was obtained.

As described above, *F*. *nucleatum subsp. nucleatum* can be specifically detected by using the above-described primer set specific to a DAACS gene of *F*. *nucleatum subsp. nucleatum.* Therefore, another aspect of the present invention can be said to be the above-described primer set for detecting *F*. *nucleatum subsp. Nucleatum,* which is specific to the DAACS gene of *F*. *nucleatum subsp. nucleatum.*

In addition, another aspect of the present invention is a kit for detecting *F*. *nucleatum subsp. nucleatum,* containing the above-described primer set specific to a DAACS gene of *F*. *nucleatum subsp. nucleatum.* The kit may further contain the above-described known reagents for performing a nucleic acid amplification reaction, such as a DNA polymerase, dNTPs, a buffer solution, a salt, and a protective agent, and may further contain the above-described labeling probe or fluorescent intercalator.

### Examples

Hereinafter, template DNAs were prepared as follows. Genomic DNA (gDNA) of the ATCC standard strain of each bacterial species was extracted using QIAamp (registered trademark) DNA Mini Kit (manufactured by Qiagen). Genomic DNA of GTC (Gifu Type Culture) strain of each bacterial species was extracted through a heat extraction method (95°C, 5 minutes). For F. canifelinum, instead of genomic DNA, an artificial gene (SEQ ID NO: 18) was used, which contains a region in the genomic DNA of the strain FDAARGO_S999 (accession: CP066022) that is highly homologous to the citrate lyase beta subunit genes having the nucleotide sequences set forth in SEQ ID NOs: 1 to 4. Each template DNA was diluted as appropriate, using a DNA diluent (5 mM tris-hydrochloric acid buffer solution containing 0.002 mg/mL salmon testis-derived single-stranded DNA (Sigma-Aldrich Co. LLC.), pH 8.0).

Hereinafter, unless otherwise stated, LAMP reaction solutions were prepared by mixing 20 µL of a master mix having the composition shown in the following table with 5 µL of a template DNA.

**[Table 2]**

| | Composition | Final concentration | Amount |
|---|---|---|---|
| LAMP reaction reagent | Tricin | 10 mM | 12.5 µL |
| | KCl | 30 mM | |
| | MgSO₄ | 8.4 mM | |
| | dNTPs | 1.7 mM each | |
| | Tween 20 | 0.5% | |
| | Bst DNApolymerase (large fragment) | 13.4 U/ reaction solution | |
| Primer mix | F3 primer | 5 pmol/ reaction solution | Appropriate amount |
| | B3 primer | 5 pmol/ reaction solution | |
| | FIP primer | 40 pmol/ reaction solution | |
| | BIP primer | 40 pmol/ reaction solution | |
| | LF primer | 20 pmol/ reaction solution | |
| | LB primer | 20 pmol/ reaction solution | |
| 5 µM SYTO 63 | | 2.5 pmol/ reaction solution | 0.5 µL |
| Purified water | | - | Remainder |
| Total | | | 20 µL |

Hereinafter, LAMP reactions were carried out using a real-time PCR instrument LightCycler (registered trademark) 480 (manufactured by Roche), CFX Maestro (manufactured by Bio-Rad Laboratories, Inc.), or a real-time turbidimeter Loopamp LA-320C (manufactured by Teramecs Co., Ltd.). Unless otherwise stated, detection of the amplification products was performed by monitoring the fluorescence intensity of SYTO (trademark) 63 Red Fluorescent Nucleic Acid Stain. The time taken for the moving average value of the fluorescence intensity to reach the threshold value a certain number of times was measured, and the first time the threshold value was exceeded was defined as the Tt value. As a no-template control (NTC), a reaction solution containing the above-described DNA diluent instead of a template DNA was used.

### <Example 1> Detection of F. nucleatum

### (1) Cross-reactivity

LAMP reactions were performed on reaction solutions containing the genomic DNA of various strains of *Fusobacterium* or an artificial gene derived from F. canifelinum (strain FDAARGO_S999), using the citrate lyase beta subunit gene (CitE)-specific primer set A1 shown in Table 3. The reactions were performed at 60°C for 90 minutes using CFX Maestro. The Tt values are shown in Table 4.

**[Table 3]**

| | SEQ ID NO | Sequence (5' to 3') |
|---|---|---|
| F3 | 5 | TGCTGCAAGATTTTTAGTTTCTG |
| B3 | 6 | TCATAATACCAGTTGCACTTTC |
| FIP | 7 | TTGGAAGTCTTACAACATTAACCCC-TAGACTATAAA ACTACTGAAACTGTTG |
| BIP | 8 | ACTCCTGATGAAATAATAGCAGTTG-GCCATAAGTAG AGTTTCTCC |
| LF | 9 | CTCTTATATCATCTGCTCCAAATGGAGT |
| LB | 10 | AACAGAAGTTGAAAAAGAAATTGGTAGAGAA |

**[Table 4]**

| Species | Bacterial strain number | Template | Number of copies | Tt value (min) | |
|---|---|---|---|---|---|
| *F. nucleatum nucleatum* | ATCC 25586 | gDNA | 1×10⁵ | 22.4 | 22.9 |
| *F. nucleatum polymorphum* | ATCC 10953 | gDNA | 1×10⁵ | 11.0 | 11.0 |
| *F. nucleatum animalis* | ATCC 51191 | gDNA | 1×10⁵ | 11.5 | 11.5 |
| *F. nucleatum vincentii* | ATCC 49256 | gDNA | 1×10⁵ | 11.0 | 11.0 |
| *F. nucleatum nucleatum* | GTC 03912 | gDNA | 1×10⁵ | 19.0 | 19.4 |
| *F. mortiferum* | GTC 21709 | gDNA | 1×10⁵ | N. D. | N. D. |
| *F. varium* | GTC 21710 | gDNA | 1×10⁵ | N. D. | N. D. |
| *F. canifelinum* | FDAARGO_S999 | SEQ ID NO: 18 | 5×10⁵ | N. D. | N. D. |
| NTC | - | - | - | N. D. | N. D. |

In the tables in the present specification, "N.D." means no detection. By using the citrate lyase beta subunit gene-specific primer set A1 shown in Table 3, it was possible to specifically detect the four subspecies of *F*. *nucleatum* without cross-reacting with other species of *Fusobacterium.*

### (2) Sensitivity

LAMP reactions were performed on reaction solutions containing 5 to 100 copies of the genomic DNA of one of the four *F*. *nucleatum* subspecies (ATCC standard strain), using the citrate lyase beta subunit gene-specific primer set A1 shown in Table 3. The reactions were performed at 60°C for 90 minutes using CFX Maestro. The Tt values are shown in Table 5.

**[Table 5]**

| | *F*. *nucleatum nucleatum* | *F*. *nucleatum polymorphum* | *F*. *nucleatum animalis* | *F*. *nucleatum vincentii* |
|---|---|---|---|---|
| | ATCC 25586 | ATCC 10953 | ATCC 51191 | ATCC 49256 |
| 100 copies | 53.3 | 15.4 | 15.4 | 15.4 |
| | 80.7 | 14.4 | 16.9 | 14.4 |
| 46 copies | 26.9 | 15.4 | 73.7 | 15.9 |
| | N. D. | 15.9 | 17.9 | 15.9 |
| 22 copies | - | 17.9 | 20.9 | 18.4 |
| | - | 15.9 | 16.4 | 17.4 |
| | - | 18.9 | 16.4 | 17.9 |
| | - | 19.4 | 19.9 | 16.9 |
| 10 copies | - | 17.4 | N.D. | 34.4 |
| | - | 16.4 | 22.9 | 23.4 |
| | - | 17.4 | N. D. | N. D. |
| | - | 18.4 | 30.4 | N. D. |
| 5 copies | - | N. D. | 68.3 | 18.4 |
| | - | 18.9 | 33.4 | 28.4 |
| | - | 18.4 | N. D. | 17.4 |
| | - | 19.4 | N. D. | N. D. |

| | | | | |
|---|---|---|---|---|
| Results for NTC were N. D. | | | | |

### (3) Combined use of two types of primer sets

LAMP reactions were performed on reaction solutions containing 10 to 100 copies of the genomic DNA of one of the four *F*. *nucleatum* subspecies (ATCC standard strain), using the citrate lyase beta subunit gene-specific primer set A1 shown in Table 3 and the dicarboxylate/amino acid:cation symporter (DAACS) gene-specific primer set B1 shown in Table 6. The reactions were performed at 60°C for 90 minutes using CFX Maestro. The Tt values are shown in Table 7.

**[Table 6]**

| | SEQ ID NO | Sequence (5' to 3') |
|---|---|---|
| F3 | 12 | TAGGAAATACTTGTAATATGAATGGT |
| B3 | 13 | GTATATCCGATATCAGTTATAGGC |
| FIP | 14 | CGAAAGAACTAATCCCATAAATACAAATTG-GTTGGA TTAGGTGTAATAAGTGT |
| BIP | 15 | GGATGTGCTGGGGTAAAAGG-AGCTGCTAAAATTGGT ATCAGTG |
| LF | 16 | AGTAATGATACAAAAGTTATTGGATAACCATAAATAT TTGAAGC |
| LB | 17 | GTCAACAGTTTTATTACAAACATTTGGAATGCC |

**[Table 7]**

| | *F*. *nucleatum nucleatum* | *F*. *nucleatum polymorphum* | *F*. *nucleatum animalis* | *F*. *nucleatum vincentii* |
|---|---|---|---|---|
| | ATCC 25586 | ATCC 10953 | ATCC 51191 | ATCC 49256 |
| 100 copies | 16.9 | 20.4 | 23.4 | 22.4 |
| | 15.9 | 20.9 | 21.9 | 21.9 |
| 46 copies | 17.4 | 21.4 | 21.9 | 24.9 |
| | 22.9 | 21.4 | 22.9 | 23.4 |
| 22 copies | 21.4 | 22.9 | 23.4 | 23.9 |
| | 18.4 | 22.9 | 28.9 | 23.4 |
| 10 copies | N.D. | 51.8 | 31.9 | 24.4 |
| | 18.9 | 37.9 | 68.3 | N.D. |

| | | | | |
|---|---|---|---|---|
| Results for NTC were N. D. | | | | |

By using the citrate lyase beta subunit gene-specific primer set A1 shown in Table 3 in combination with the DAACS gene-specific primer set B1 shown in Table 6, the detection sensitivity of *F*. *nucleatum subsp. nucleatum* was improved, compared to using the citrate lyase beta subunit gene-specific primer set A1 alone (Table 5). The detection sensitivity of subspecies other than *F*. *nucleatum subsp. nucleatum* was equivalent to using the citrate lyase beta subunit gene-specific primer set A1 alone.

### <Example 2> Detection of F. nucleatum subsp. nucleatum

### (1) Cross-reactivity

LAMP reactions were performed on reaction solutions containing the genomic DNA of various strains of *Fusobacterium,* using the DAACS gene-specific primer set B1 shown in Table 6. The LAMP reactions were performed at 65°C for 90 minutes using CFX Maestro. The Tt values are shown in Table 8.

**[Table 8]**

| Species | Bacterial strain number | Template | Number of copies | Tt value (min) | |
|---|---|---|---|---|---|
| *F. nucleatum nucleatum* | ATCC 25586 | gDNA | 1×10⁵ | 10.9 | 10.4 |
| *F. nucleatum polymorphum* | ATCC 10953 | gDNA | 1×10⁵ | N. D. | N. D. |
| *F. nucleatum animalis* | ATCC 51191 | gDNA | 1×10⁵ | N. D. | N. D. |
| *F. nucleatum vincentii* | ATCC 49256 | gDNA | 1×10⁵ | N. D. | N. D. |
| *F. nucleatum nucleatum* | GTC 03912 | gDNA | 1×10⁵ | N. D. | N. D. |
| *F. mortiferum* | GTC 21709 | gDNA | 1×10⁵ | N. D. | N. D. |
| *F. varium* | GTC 21710 | gDNA | 1×10⁵ | N. D. | N. D. |
| NTC | - | - | - | N. D. | N. D. |

By using the DAACS gene-specific primer set B1 shown in Table 6, it was possible to specifically detect *F*. *nucleatum subsp. nucleatum* without cross-reacting with other subspecies and species.

### (2) Sensitivity

LAMP reactions were performed on reaction solutions containing 5 to 80 copies of the genomic DNA of *F*. *nucleatum subsp. nucleatum* (strain ATCC 25586), using the DAACS gene-specific primer set B1 shown in Table 6. The reactions were performed at 65°C for 90 minutes using LightCycler (registered trademark) 480. The Tt values are shown in Table 9.

**[Table 9]**

| | Tt value (min) | | | |
|---|---|---|---|---|
| 80 copies | 15.4 | 17.3 | 16.5 | 14.8 |
| 40 copies | 25.8 | 16.7 | 16.9 | 17.7 |
| 20 copies | 16.5 | N.D. | 19.4 | 20.7 |
| 10 copies | N. D. | 22.0 | N. D. | N. D. |
| 5 copies | N. D. | N. D. | 30.7 | N. D. |
| NTC | N. D. | N. D. | N. D. | N. D. |

### (3) Comparison with primer sets specific to other regions

LAMP reactions were performed on reaction solutions containing 100 or 1,000 copies of the genomic DNA of *F*. *nucleatum subsp. nucleatum* (strain ATCC 25586), using the DAACS gene-specific primer set B1 shown in Table 6 or any of the comparative primer sets 1 to 3 shown in Table 10. The comparative primer sets 1 to 3 are primer sets specific to the "cell filamentation protein Fic" gene having a similarly high degree of sequence identity among strains of *F*. *nucleatum subsp. nucleatum* as the DAACS gene. The reactions were performed at 65°C for 180 minutes using Loopamp LA-320C. However, SYTO (trademark) 63 Red Fluorescent Nucleic Acid Stain was not added to the reaction solutions, and the detection of the amplification product was performed by monitoring the turbidity of the reaction solution. The time until the turbidity reaches 0.1 was measured (Tt value). The Tt values are shown in Table 11.

**[Table 10]**

| | | SEQ ID NO | Sequence (5' to 3') |
|---|---|---|---|
| Comparative primer set 1 | F3 | 19 | AATACTACGATTCATTGACAAGA |
| | B3 | 20 | GTTAATTCCTCAAGAATACCGATA |
| | FIP | 21 | GTTTGTGCTGTAATTATAACACCTTTTTAAA- GTTAGAGAAAATAATGATATGATTTCATGG |
| | BIP | 22 | TGACTGTAAAAAATTATGAAGAAAAAGTTTC T-CGCTTTAATATATTATTAACTGTTGGA |
| Comparative primer set 2 | F3 | 23 | AGAGAAAATAATGATATGATTTCATGG |
| | B3 | 24 | CCTTCTATAAATATTGAAAGATATTCCATT |
| | FIP | 25 | AGAAACTTTTTCTTCATAATTTTTTACAGTCA- AAAGGTGTTATAATTACAGCACA |
| | BIP | 26 | GTTTTCCAACAGTTAATAATATATTAAAGCGT -TCTTTTATAATTGGTTAATTCCTCAAG |
| Comparative primer set 3 | F3 | 27 | GGGAGATTGATGATACCTCT |
| | B3 | 28 | CTTTTTCTTCATAATTTTTTACAGTCAT |
| | FIP | 29 | GAATCGTAGTATTCTGTTCTATTTTTTTCAAA- TTGCTAGATAAAATATATTGAATAAACCATG |
| | BIP | 30 | ATTGACAAGAGTTAGAGAAAATAATGATATG A-TCTTAGCTGTTTGTGCTGT |

**[Table 11]**

| | Comparative primer set | | | DAACS gene-specific primer set | |
|---|---|---|---|---|---|
| | 1 | 2 | 3 | Without LF and LB | With LF and LB |
| 1,000 copies | N. D. | 100.8 | 140.2 | 51.0 | 21.8 |
| | N. D. | 124.0 | 130.8 | 51.7 | 21.8 |
| 100 copies | N. D. | 164.0 | N. D. | 64.8 | 25.4 |
| | N. D. | 160.8 | 155.8 | 63.1 | 26.9 |
| NTC | N. D. | N. D. | N. D. | N. D. | N. D. |
| | N. D. | N. D. | N. D. | N. D. | N. D. |

The comparative primer sets 1 to 3 specific to the cell filamentation protein Fic gene failed to detect *F. nucleatum subsp. nucleatum,* or required longer time to detect and were less sensitive compared to using the DAACS-specific primer set B1 shown in Table 6.

### <Example 3> Detection of F. nucleatum

### (1) Cross-reactivity

LAMP reactions were performed on reaction solutions containing 1×10⁵ copies of the genomic DNA of various strains of *Fusobacterium* or an artificial gene derived from F. canifelinum (strain FDAARGO_S999), using CitE-specific primer sets A2 to A4 shown in Table 12. The reactions were performed at 60°C for 140 minutes using CFX Maestro. The Tt values are shown in Table 13.

**[Table 12]**

| | | SEQ ID NO | Sequence (5' to 3') |
|---|---|---|---|
| Primer set A2 | F3 | 31 | GCAATTAGAGATAGATTAAGAAGAAC |
| | B3 | 32 | CTCTTATATCATCTGCTCCAAAT |
| | FIP | 33 | TTTACACTAGTAGCATCTTCTAAGTC-CTAC CTGGTAATAATCCATCAATG |
| | BIP | 34 | CAAAAAGATGCTGCAAGATTTTTAGTTTCT G-CTAAACCATTTACYCTTACAACAG |
| | LF | 35 | ATCTGGTCCATATATATAAGCATCTGTAAT |
| | LB | 36 | GCTTTAAAAACAATAGATTATAAAACTAC TGAAA |
| Primer set A3 | F3 | 37 | GCAGATGATATAAGAGCTGTTG |
| | B3 | 38 | TCTTGCAGCAAGCACAAT |
| | FIP | 39 | CCATTAATCTTTTGCTAGCAAGAGCAA-GA GTTAATGTTGTAAGACTTCCAAAAA |
| | BIP | 40 | AGGAGCAGAAGACTATGTTACTAACT-CTT GCATAATATAATTCCCAACC |
| | LF | 41 | TTTCTTTAACATTCATAATACCAGTTGCAC |
| | LB | 42 | TAACTTAAAGACTTCAAGAAGTAAACAT |
| Primer set A4 | F3 | 43 | TGCTTTGATACTGTTTATTCAGA |
| | B3 | 44 | CCATCAACAGATATAACTCCTGA |
| | FIP | 45 | TGTATACAAGATTTACCATCAAATCC-GTA AATAACCTAGATGGTTTCAGAC |
| | BIP | 46 | TTAGAATAGTTCATGAAATTTATACACCAA -ACCCTTAGCTTCAGCTTC |
| | LF | 47 | CAAATCCTAAATCTTTAATAAATTGAACTT CTT |
| | LB | 48 | AAAAAGAAATTGAAAAATCAATCAGAATT ATAAAT |

**[Table 13]**

| Species | Bacterial strain number | Template | Primer set | | |
|---|---|---|---|---|---|
| | | | A2 | A3 | A4 |
| *F. nucleatum nucleatum* | GTC 03912 | gDNA | 13.5 | 18.4 | 24.5 |
| *F. mortiferum* | GTC 21709 | gDNA | N. D. | N. D. | N. D. |
| *F. varium* | GTC 21710 | gDNA | N. D. | N. D. | N. D. |
| *F. canifelinum* | FDAARGO_S999 | SEQ ID NO: 18 | N. D. | N. D. | N. D. |
| NTC | - | - | N. D. | N. D. | N. D. |

By using the CitE-specific primer sets A2 to A4, it was possible to specifically detect *F. nucleatum* without cross-reacting with other species of *Fusobacterium.*

### (2) Sensitivity

LAMP reactions were performed on reaction solutions containing 22 to 100 copies of the genomic DNA of one of the four *F*. *nucleatum* subspecies (ATCC standard strain), using the CitE-specific primer sets A2 to A4. The reactions were performed at 60°C for 120 minutes using CFX Maestro. The Tt values are shown in Table 14.

**[Table 14]**

| | *F. nucleatum* subspecies | Primer set | | |
|---|---|---|---|---|
| | | A2 | A3 | A4 |
| 100 copies | *Nucleatum* | 18.4 | 26.3 | 35.5 |
| | *Polymorphum* | 29.4 | 21.4 | 23.9 |
| | *Animalis* | 21.4 | 23.3 | 20.2 |
| | *Vincentii* | 18.4 | 23.9 | 31.2 |
| 46 copies | *Nucleatum* | 39.2 | N. D. | 35.5 |
| | *Polymorphum* | 27.5 | 22.6 | 24.5 |
| | *Animalis* | 20.2 | 21.4 | 20.2 |
| | *Vincentii* | 18.4 | 23.9 | 78.9 |
| 22 copies | *Nucleatum* | N. D. | 27.5 | N. D. |
| | *Polymorphum* | 37.3 | 28.2 | 24.5 |
| | *Animalis* | N. D. | N. D. | 23.9 |
| | *Vincentii* | N. D. | 24.5 | 79.6 |
| NTC | - | N. D. | N. D. | N. D. |
| | - | N. D. | N. D. | N. D. |

### <Example 4> Detection of F. nucleatum subsp. nucleatum

### (1) Cross-reactivity

LAMP reactions were performed on reaction solutions containing 1×10⁵ copies of the genomic DNA of various strains of *Fusobacterium,* using DAACS gene-specific primer sets B2 to B4 shown in Table 15. The LAMP reactions were performed at 65°C for 90 minutes using CFX Maestro. The Tt values are shown in Table 16.

**[Table 15]**

| | | SEQ ID NO | Sequence (5' to 3') |
|---|---|---|---|
| Primer set B2 | F3 | 49 | CAATTAAGAACAGTTGGAATGAGAA |
| | B3 | 50 | CCCATTAATAGAGCAAAAAAGATAATCTG T |
| | FIP | 51 | TAACTCCCTTTCCTGGTTGAGTA-TCTATCT TGCTTCCTCAACTGTTTC |
| | BIP | 52 | TAGGTACTGAAGTTGGAAAAGATGTT-TGT ATTTCCTGTTGCTAATGC |
| | LF | 53 | AACAAAGCAATTAAAACTCCTAATATTGC |
| | LB | 54 | ATTGAAAATTTAATTGGCTGGGTCCC |
| Primer set B3 | F3 | 55 | GCTGATTTAACTATTTCTTTATCTGG |
| | B3 | 56 | GTATTTCCTAATGGTAAAGTAAAACC |
| | FIP | 57 | TCCACTTTCCTTTGTGAATAATTTTATTGC- CAAGTATTAAACATGATACTAACTGTCTAC |
| | BIP | 58 | TGTTACTCCTGCAATGATTGTAGCT-TTCAG GAACTCCTAATTCTTCA |
| | LF | 59 | AGGATAAACTATAAATATTATGAACAATA AAACTAT |
| | LB | 60 | CATCTTCTGCAGCAACTTTACCA |
| Primer set B4 | F3 | 61 | CCTCAACAACATCTTCTGCA |
| | B3 | 62 | TGTTGACATAACTATTCCTGCA |
| | FIP | 63 | CCATTCATATTACAAGTATTTCCTAATGGT- CTTTACCAGTTTCTTTAAGATGTGCA |
| | BIP | 64 | TGGATTAGGTGTAATAAGTGTATTTGCT-A ACCGAAAGAACTAATCCCATA |
| | LF | 65 | AAAGTAAAACCAAATATATTTTCAGGAAC TCC |
| | LB | 66 | ATGGTTATCCAATAACTTTTGTATCATTAC TTCA |

**[Table 16]**

| Species | Bacterial strain number | Primer set | | |
|---|---|---|---|---|
| | | B2 | B3 | B4 |
| *F. nucleatum polymorphum* | ATCC10953 | N. D. | N. D. | N. D. |
| *F. nucleatum animalis* | ATCC51191 | N. D. | N. D. | N. D. |
| *F. nucleatum vincentii* | ATCC49256 | N. D. | N. D. | N. D. |
| *F. nucleatum nucleatum* | GTC03912 | 15.3 | 31.8 | 11.6 |
| *F. mortiferum* | GTC21709 | N. D. | N. D. | N. D. |
| *F. varium* | GTC21710 | N. D. | N. D. | N. D. |
| NTC | - | N. D. | N. D. | N. D. |

By using the DAACS gene-specific primer sets B2 to B4, it was possible to specifically detect *F. nucleatum subsp. nucleatum* without cross-reacting with other subspecies and species.

### (2) Sensitivity

LAMP reactions were performed on reaction solutions containing 22 to 1,000 copies of the genomic DNA of *F. nucleatum subsp. nucleatum* (strain ATCC 25586), using the DAACS gene-specific primer sets B2 to B4. The reactions were performed at 65°C for 90 minutes using LightCycler (registered trademark) 480. The Tt values are shown in Table 17.

**[Table 17]**

| | Primer set | | |
|---|---|---|---|
| | B2 | B3 | B4 |
| 1,000 copies | 9.0 | 19.9 | 9.3 |
| 100 copies | 10.6 | 22.0 | 10.6 |
| | 11.2 | 24.2 | 11.6 |
| 46 copies | 11.6 | 25.0 | 11.4 |
| | 12.1 | 24.2 | 15.1 |
| 22 copies | 11.2 | 28.1 | 11.9 |
| | 14.9 | 26.7 | 12.9 |
| NTC | N. D. | N. D. | N. D. |

### <Example 5> Detection of F. nucleatum

### (1) Cross-reactivity

PCR reactions were performed on reaction solutions containing the genomic DNA of various strains of *Fusobacterium* or an artificial gene derived from F. canifelinum (strain FDAARGO_S999), using the CitE-specific primer set A5 shown in Table 18.

**[Table 18]**

| | SEQ ID NO | Sequence (5' to 3') |
|---|---|---|
| Forward | 67 | TGATGTTTCTACCTGGTAATAATCCATC |
| Reverse | 68 | TTGAACTTCTTGTCTGAAACCATC |

PCR reaction solutions were prepared by mixing 20 µL of a master mix having the composition shown in the following table with 5 µL of a template DNA.

**[Table 19]**

| Composition | Amount |
|---|---|
| Quick Taq^{®} HS DyeMix (manufactured by Toyobo Co., Ltd.) | 12.5 µL |
| 10 µM SYBR Green I | 0.25 µL |
| 100 µM Forward primer | 0.25 µL |
| 100 µM Reverse primer | 0.25 µL |
| Purified water | Remainder |
| Total | 20 µL |

PCR reactions were performed using CFX Maestro (manufactured by Bio-Rad Laboratories, Inc.) (95°C for 2 minutes, followed by 45 cycles of 95°C for 15 seconds and 60°C for 1 minute). Detection of the amplification products were performed by monitoring the fluorescence intensity of SYBR (trademark) Green I Nucleic Acid Gel Stain. The time taken for the moving average value of the fluorescence intensity to reach the threshold value a certain number of times was measured, and the first time the threshold value was exceeded was defined as the Tt value. As an NTC, a reaction solution containing the above-described DNA diluent instead of a template DNA was used. The Tt values are shown in Table 20.

**[Table 20]**

| Species | Bacterial strain number | Template | Tt value (min) |
|---|---|---|---|
| *F. nucleatum polymorphum* | ATCC 10953 | gDNA | 28.5 |
| *F. canifelinum* | FDAARGO_S999 | SEQ ID NO: 18 | N. D. |
| NTC | - | - | N. D. |

It was shown that by using the CitE-specific primer set A5, it was possible to specifically detect *F. nucleatum* by PCR without cross-reacting with F. canifelinum, which has a region with the highest homology to CitE of *F. nucleatum.*

## Claims

1. A method for detecting *Fusobacterium nucleatum* in a sample, comprising:
a step of performing a nucleic acid amplification reaction on a DNA derived from the sample using a primer set specific to a citrate lyase beta subunit gene of *Fusobacterium nucleatum.*

2. The method according to claim 1, wherein the citrate lyase beta subunit gene consists of the nucleotide sequence set forth in any one of SEQ ID NOs: 1 to 4 or a nucleotide sequence having 90% or more sequence identity to the nucleotide sequence set forth in any one of SEQ ID NOs: 1 to 4.

3. The method according to claim 2, wherein the primer set is a primer set specific to
a region from a 141st to 415th base,
a region from a 4th to a 253rd base,
a region from a 238th to a 561st base,
a region from a 577th to a 806th base, or
a region from a 32nd to a 636th base in the nucleotide sequence set forth in any one of SEQ ID NOs: 1 to 4,
or a region having 90% or more sequence identity to any of these regions.

4. The method according to any one of claims 1 to 3, wherein the nucleic acid amplification reaction is a loop-mediated isothermal amplification reaction or a polymerase chain reaction.

5. The method according to claim 4,
wherein the nucleic acid amplification reaction is a loop-mediated isothermal amplification reaction, and
wherein the primer set is
(a1) a primer set comprising an F3 primer consisting of the nucleotide sequence set forth in SEQ ID NO: 5, a B3 primer consisting of the nucleotide sequence set forth in SEQ ID NO: 6, an FIP primer consisting of the nucleotide sequence set forth in SEQ ID NO: 7, and a BIP primer consisting of the nucleotide sequence set forth in SEQ ID NO: 8,
(a2) a primer set comprising an F3 primer consisting of the nucleotide sequence set forth in SEQ ID NO: 31, a B3 primer consisting of the nucleotide sequence set forth in SEQ ID NO: 32, an FIP primer consisting of the nucleotide sequence set forth in SEQ ID NO: 33, and a BIP primer consisting of the nucleotide sequence set forth in SEQ ID NO: 34,
(a3) a primer set comprising an F3 primer consisting of the nucleotide sequence set forth in SEQ ID NO: 37, a B3 primer consisting of the nucleotide sequence set forth in SEQ ID NO: 38, an FIP primer consisting of the nucleotide sequence set forth in SEQ ID NO: 39, and a BIP primer consisting of the nucleotide sequence set forth in SEQ ID NO: 40, or
(a4) a primer set comprising an F3 primer consisting of the nucleotide sequence set forth in SEQ ID NO: 43, a B3 primer consisting of the nucleotide sequence set forth in SEQ ID NO: 44, an FIP primer consisting of the nucleotide sequence set forth in SEQ ID NO: 45, and a BIP primer consisting of the nucleotide sequence set forth in SEQ ID NO: 46.

6. The method according to claim 5,
wherein the primer set of (a1) further comprises an LF primer consisting of the nucleotide sequence set forth in SEQ ID NO: 9 and an LB primer consisting of the nucleotide sequence set forth in SEQ ID NO: 10,
wherein the primer set of (a2) further comprises an LF primer consisting of the nucleotide sequence set forth in SEQ ID NO: 35 and an LB primer consisting of the nucleotide sequence set forth in SEQ ID NO: 36,
wherein the primer set of (a3) further comprises an LF primer consisting of the nucleotide sequence set forth in SEQ ID NO: 41 and an LB primer consisting of the nucleotide sequence set forth in SEQ ID NO: 42, and
wherein the primer set of (a4) further comprises an LF primer consisting of the nucleotide sequence set forth in SEQ ID NO: 47 and an LB primer consisting of the nucleotide sequence set forth in SEQ ID NO: 48.

7. The method according to claim 4,
wherein the nucleic acid amplification reaction is a polymerase chain reaction, and
wherein the primer set is (a5) a primer set comprising a forward primer consisting of the nucleotide sequence set forth in SEQ ID NO: 67 and a reverse primer consisting of the nucleotide sequence set forth in SEQ ID NO: 68.

8. The method according to any one of claims 1 to 3, wherein, in the step of performing a nucleic acid amplification reaction, a primer set specific to a dicarboxylate/amino acid:cation symporter gene of *Fusobacterium nucleatum subsp. nucleatum* is further used in conjunction with the primer set specific to a citrate lyase beta subunit gene of *Fusobacterium nucleatum.*

9. A primer set specific to a citrate lyase beta subunit gene of *Fusobacterium nucleatum* for detecting *Fusobacterium nucleatum.*

10. The primer set according to claim 9, wherein the citrate lyase beta subunit gene consists of the nucleotide sequence set forth in any one of SEQ ID NOs: 1 to 4 or a nucleotide sequence having 90% or more sequence identity to the nucleotide sequence set forth in any one of SEQ ID NOs: 1 to 4.

11. The primer set according to claim 10, which is specific to
a region from a 141st to a 415th base,
a region from a 4th to a 253rd base,
a region from a 238th to a 561st base,
a region from a 577th to a 806th base, or
a region from a 32nd to a 636th base in the nucleotide sequence set forth in any one of SEQ ID NOs: 1 to 4,
or a region having 90% or more sequence identity to any of these regions.

12. The primer set according to any one of claims 9 to 11, which is for a loop-mediated isothermal amplification reaction or a polymerase chain reaction.

13. The primer set according to claim 12, which is for a loop-mediated isothermal amplification reaction and is
(a1) a primer set comprising an F3 primer consisting of the nucleotide sequence set forth in SEQ ID NO: 5, a B3 primer consisting of the nucleotide sequence set forth in SEQ ID NO: 6, an FIP primer consisting of the nucleotide sequence set forth in SEQ ID NO: 7, and a BIP primer consisting of the nucleotide sequence set forth in SEQ ID NO: 8,
(a2) a primer set comprising an F3 primer consisting of the nucleotide sequence set forth in SEQ ID NO: 31, a B3 primer consisting of the nucleotide sequence set forth in SEQ ID NO: 32, an FIP primer consisting of the nucleotide sequence set forth in SEQ ID NO: 33, and a BIP primer consisting of the nucleotide sequence set forth in SEQ ID NO: 34,
(a3) a primer set comprising an F3 primer consisting of the nucleotide sequence set forth in SEQ ID NO: 37, a B3 primer consisting of the nucleotide sequence set forth in SEQ ID NO: 38, an FIP primer consisting of the nucleotide sequence set forth in SEQ ID NO: 39, and a BIP primer consisting of the nucleotide sequence set forth in SEQ ID NO: 40, or
(a4) a primer set comprising an F3 primer consisting of the nucleotide sequence set forth in SEQ ID NO: 43, a B3 primer consisting of the nucleotide sequence set forth in SEQ ID NO: 44, an FIP primer consisting of the nucleotide sequence set forth in SEQ ID NO: 45, and a BIP primer consisting of the nucleotide sequence set forth in SEQ ID NO: 46.

14. The primer set according to claim 13,
wherein the primer set of (a1) further comprises an LF primer consisting of the nucleotide sequence set forth in SEQ ID NO: 9 and an LB primer consisting of the nucleotide sequence set forth in SEQ ID NO: 10,
wherein the primer set of (a2) further comprises an LF primer consisting of the nucleotide sequence set forth in SEQ ID NO: 35 and an LB primer consisting of the nucleotide sequence set forth in SEQ ID NO: 36,
wherein the primer set of (a3) further comprises an LF primer consisting of the nucleotide sequence set forth in SEQ ID NO: 41 and an LB primer consisting of the nucleotide sequence set forth in SEQ ID NO: 42, and
wherein the primer set of (a4) further comprises an LF primer consisting of the nucleotide sequence set forth in SEQ ID NO: 47 and an LB primer consisting of the nucleotide sequence set forth in SEQ ID NO: 48.

15. The primer set according to claim 12, which is for a polymerase chain reaction and is (a5) a primer set comprising a forward primer consisting of the nucleotide sequence set forth in SEQ ID NO: 67 and a reverse primer consisting of the nucleotide sequence set forth in SEQ ID NO: 68.

16. A method for detecting *Fusobacterium nucleatum subsp. nucleatum* in a sample, comprising:
a step of performing a nucleic acid amplification reaction on a DNA derived from the sample using a primer set specific to a dicarboxylate/amino acid:cation symporter gene of *Fusobacterium nucleatum subsp. nucleatum.*

17. The method according to claim 16, wherein the dicarboxylate/amino acid:cation symporter gene consists of the nucleotide sequence set forth in SEQ ID NO: 11 or a nucleotide sequence having 90% or more sequence identity to the nucleotide sequence set forth in SEQ ID NO: 11.

18. The method according to claim 17, wherein the primer set is a primer set specific to
a region from a 860th to a 1121st base,
a region from a 196th to a 449th base,
a region from a 580th to a 869th base, or
a region from a 761st to a 1038th base in the nucleotide sequence set forth in SEQ ID NO: 11,
or a region having 90% or more sequence identity to any of these regions.

19. The method according to any one of claims 16 to 18, wherein the nucleic acid amplification reaction is a loop-mediated isothermal amplification reaction.

20. The method according to claim 19, wherein the primer set is
(b1) a primer set comprising an F3 primer consisting of the nucleotide sequence set forth in SEQ ID NO: 12, a B3 primer consisting of the nucleotide sequence set forth in SEQ ID NO: 13, an FIP primer consisting of the nucleotide sequence set forth in SEQ ID NO: 14, and a BIP primer consisting of the nucleotide sequence set forth in SEQ ID NO: 15,
(b2) a primer set comprising an F3 primer consisting of the nucleotide sequence set forth in SEQ ID NO: 49, a B3 primer consisting of the nucleotide sequence set forth in SEQ ID NO: 50, an FIP primer consisting of the nucleotide sequence set forth in SEQ ID NO: 51, and a BIP primer consisting of the nucleotide sequence set forth in SEQ ID NO: 52,
(b3) a primer set comprising an F3 primer consisting of the nucleotide sequence set forth in SEQ ID NO: 55, a B3 primer consisting of the nucleotide sequence set forth in SEQ ID NO: 56, an FIP primer consisting of the nucleotide sequence set forth in SEQ ID NO: 57, and a BIP primer consisting of the nucleotide sequence set forth in SEQ ID NO: 58, or
(b4) a primer set comprising an F3 primer consisting of the nucleotide sequence set forth in SEQ ID NO: 61, a B3 primer consisting of the nucleotide sequence set forth in SEQ ID NO: 62, an FIP primer consisting of the nucleotide sequence set forth in SEQ ID NO: 63, and a BIP primer consisting of the nucleotide sequence set forth in SEQ ID NO: 64.

21. The method according to claim 20,
wherein the primer set of (b1) further comprises an LF primer consisting of the nucleotide sequence set forth in SEQ ID NO: 16 and an LB primer consisting of the nucleotide sequence set forth in SEQ ID NO: 17,
wherein the primer set of (b2) further comprises an LF primer consisting of the nucleotide sequence set forth in SEQ ID NO: 53 and an LB primer consisting of the nucleotide sequence set forth in SEQ ID NO: 54,
wherein the primer set of (b3) further comprises an LF primer consisting of the nucleotide sequence set forth in SEQ ID NO: 59 and an LB primer consisting of the nucleotide sequence set forth in SEQ ID NO: 60, and
wherein the primer set of (b4) further comprises an LF primer consisting of the nucleotide sequence set forth in SEQ ID NO: 65 and an LB primer consisting of the nucleotide sequence set forth in SEQ ID NO: 66.

22. A primer set specific to a dicarboxylate/amino acid:cation symporter gene of *Fusobacterium nucleatum subsp. nucleatum* for detecting *Fusobacterium nucleatum subsp. nucleatum.*

23. The primer set according to claim 22, wherein the dicarboxylate/amino acid:cation symporter gene consists of the nucleotide sequence set forth in SEQ ID NO: 11 or a nucleotide sequence having 90% or more sequence identity to the nucleotide sequence set forth in SEQ ID NO: 11.

24. The primer set according to claim 23, which is specific to
a region from a 860th to a 1121st base,
a region from a 196th to a 449th base,
a region from a 580th to a 869th base, or
a region from a 761st to a 1038th base in the nucleotide sequence set forth in SEQ ID NO: 11,
or a region having 90% or more sequence identity to any of these regions.

25. The primer set according to any one of claims 22 to 24, which is a primer set for a loop-mediated isothermal amplification reaction.

26. The primer set according to claim 25, which is
(b1) a primer set comprising an F3 primer consisting of the nucleotide sequence set forth in SEQ ID NO: 12, a B3 primer consisting of the nucleotide sequence set forth in SEQ ID NO: 13, an FIP primer consisting of the nucleotide sequence set forth in SEQ ID NO: 14, and a BIP primer consisting of the nucleotide sequence set forth in SEQ ID NO: 15,
(b2) a primer set comprising an F3 primer consisting of the nucleotide sequence set forth in SEQ ID NO: 49, a B3 primer consisting of the nucleotide sequence set forth in SEQ ID NO: 50, an FIP primer consisting of the nucleotide sequence set forth in SEQ ID NO: 51, and a BIP primer consisting of the nucleotide sequence set forth in SEQ ID NO: 52,
(b3) a primer set comprising an F3 primer consisting of the nucleotide sequence set forth in SEQ ID NO: 55, a B3 primer consisting of the nucleotide sequence set forth in SEQ ID NO: 56, an FIP primer consisting of the nucleotide sequence set forth in SEQ ID NO: 57, and a BIP primer consisting of the nucleotide sequence set forth in SEQ ID NO: 58, or
(b4) a primer set comprising an F3 primer consisting of the nucleotide sequence set forth in SEQ ID NO: 61, a B3 primer consisting of the nucleotide sequence set forth in SEQ ID NO: 62, an FIP primer consisting of the nucleotide sequence set forth in SEQ ID NO: 63, and a BIP primer consisting of the nucleotide sequence set forth in SEQ ID NO: 64.

27. The primer set according to claim 26,
wherein the primer set of (b1) further comprises an LF primer consisting of the nucleotide sequence set forth in SEQ ID NO: 16 and an LB primer consisting of the nucleotide sequence set forth in SEQ ID NO: 17,
wherein the primer set of (b2) further comprises an LF primer consisting of the nucleotide sequence set forth in SEQ ID NO: 53 and an LB primer consisting of the nucleotide sequence set forth in SEQ ID NO: 54,
wherein the primer set of (b3) further comprises an LF primer consisting of the nucleotide sequence set forth in SEQ ID NO: 59 and an LB primer consisting of the nucleotide sequence set forth in SEQ ID NO: 60, and
wherein the primer set of (b4) further comprises an LF primer consisting of the nucleotide sequence set forth in SEQ ID NO: 65 and an LB primer consisting of the nucleotide sequence set forth in SEQ ID NO: 66.
